# EUROPEAN PATENT APPLICATION

(11) **EP 4 201 947 A1**
(43) Date of publication of application: **28.06.2023**
(21) Application number: 21216879.3
(22) Date of filing: 22.12.2021
(51) Int. Cl.: C07H 1/00, C07H 21/00

(54) **PROCESS FOR THE BACKBONE DEPROTECTION OF OLIGONUCLEOTIDES CONTAINING A TERMINAL ALKYL PHOSPHONATE GROUP**

(71) Applicant: F. Hoffmann-La Roche AG, 4070 Basel (CH)
(72) Inventor: The designation of the inventor has not yet been filed
(74) Representative: Rauber, Beat

(57) **Abstract**

The invention comprises a process for the production of a linear P-linked oligonucleotide bearing an alkyl phosphonate group at the 5'-terminus of the formula 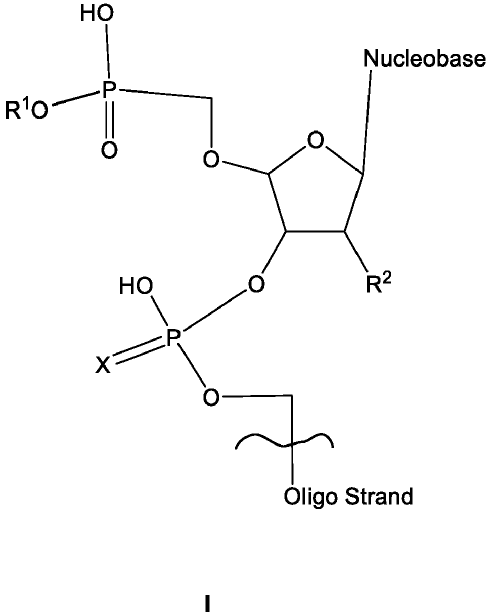 wherein R¹ is a C₁₋₄-alkyl group, R² is fluoro, hydroxyl or C₁₋₄-alkoxy, X is sulfur or oxygen, the term Nucleobase stands for an optionally modified adenine, cytosine, guanine or uracil and the term oligo strand stands for the remaining P-linked oligo nucleotide strand, and wherein the oligo strand comprises at least one uracil nucleobase.

The process is performed under conditions that the level of alkyl transfer impurities and the level of cyanoethyl (CNET) impurities in the linear P-linked oligonucleotide of formula I, is substantially reduced.

## Description

The invention relates to a novel process for the production of a linear P-linked oligonucleotide bearing an alkylphosphonate group at the 5'-terminus of the formula I 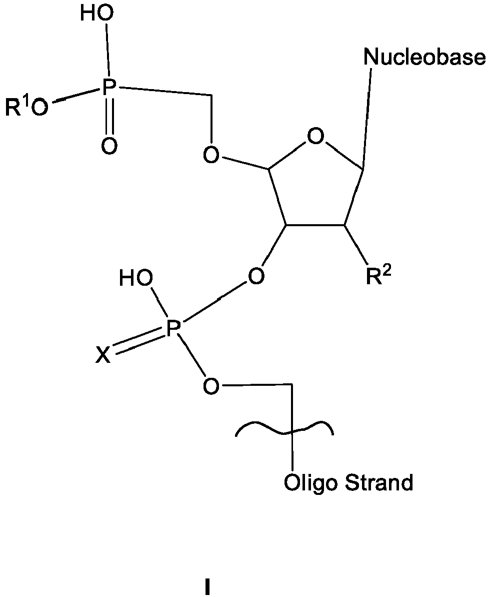 wherein R¹ is a C₁₋₄-alkyl group, R² is fluoro, hydroxyl or C₁₋₄-alkoxy, X is sulfur or oxygen, the term Nucleobase stands for an optionally modified adenine, cytosine, guanine or uracil and the term oligo strand stands for the remaining P-linked oligo nucleotide strand and wherein the oligo strand comprises at least one uracil nucleobase.

P-linked oligonucleotides bearing an alkylphosphonate group at the 5'-terminus of the formula I are therapeutically valuable compounds which may form antisense strands, can target various antigens and thereby block their expression. As an example P-linked oligonucleotides bearing an alkylphosphonate group at the 5'-terminus of the formula I may form antisense strands targeting HBV surface antigens (HBsAg) and effecting knock down of HBsAg expression and accordingly may be useful to treat HBV infections (International Publication WO 2019/079781).

The process comprises the removal of the cyanoethyl group and one C₁₋₄ alkyl group from the oligonucleotide compound of formula II 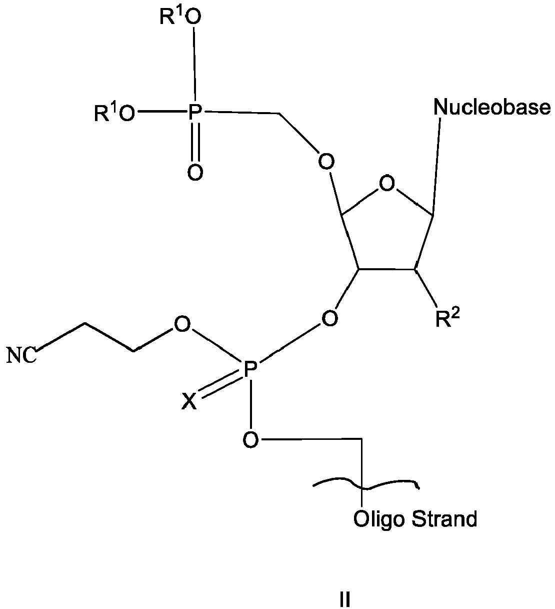 wherein R¹, R², X, the term Nucleobase and the term oligo strand is as above, with a nucleophilic organic base in the presence of an organic solvent.

The oligonucleotide synthesis in principle is a stepwise addition of nucleoside residues to the 5'-terminus of the growing chain until the desired sequence is assembled.

As a rule, each addition is referred to as a synthetic cycle and in principle consists of the chemical reactions
a₁) de-blocking the 5'protected hydroxyl group on the solid support,
a₂) coupling the first nucleoside as activated phosphoramidite with the free hydroxyl group on the solid support,
a₃) oxidizing or sulfurizing the respective P-linked nucleoside to form the respective phosphodiester (P=O) or the respective phosphorothioate (P=S);
a₄) optionally, capping any unreacted hydroxyl groups on the solid support,
a₅) de-blocking the 5' hydroxyl group of the first nucleoside attached to the solid support;
a₆) coupling the second nucleoside as activated phosphoramidite to form the respective P-O linked dimer;
a₇) oxidizing or sulfurizing the respective P-O linked dinucleoside to form the respective phosphodiester (P=O) or the respective phosphorothioate (P=S);
a₈) optionally, capping any unreacted 5' hydroxyl groups;
a₉) repeating the previous steps as to as until the desired sequence is assembled.

The reaction sequence may alternatively start with de-blocking of the 5' protected hydroxyl group of the nucleoside which is preloaded on the solid support. The subsequent steps follow the sequence as outlined above.

Finally, the assembled oligonucleotide is treated with a base to remove the cyanoethyl protecting group and cleaved from the solid support and subsequent downstream processing and purification methods provide the desired pure oligonucleotide.

The backbone deprotection i.e. the removal of the cyanoethyl group from the phosphodiester or phosphorothioate linkage is standard in principle and is well known in the art.

The US Patent 6,887,990 illustrates the standard procedure and discloses the removal of cyanoethyl groups with an amine such as diethylamine in an acetonitrile solution.

The removal of one alkyl group from an oligonucleotide compound of formula II has been described with concentrated ammonia at 55°C in the PCT International Publication WO 2018/045317.

It has been observed that in the course of the process the alkyl group has a tendency to alkylate uracil nucleobases in the neighborhood of the alkylphosphonate nucleotide, which results in so called alkyl transfer impurities which cannot be significantly depleted in the subsequent downstream processing steps. An oligo structure with a methylated uracil nucleobase is shown in formula III. R² has the meaning as outlined before.

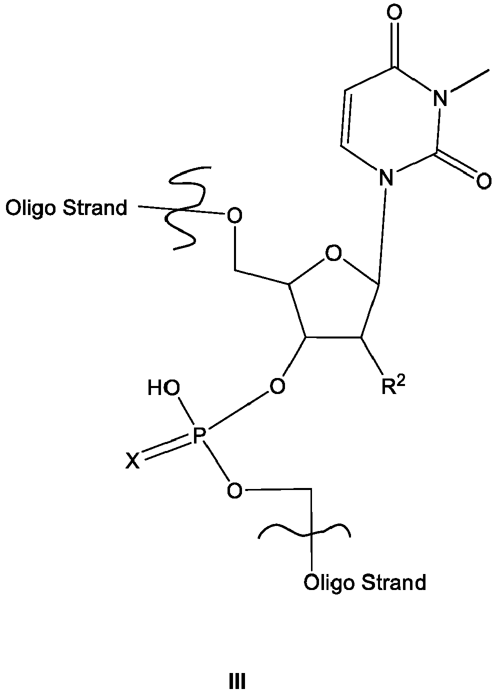

Object of the present invention therefore was to further improve the process for the backbone deprotection, i.e. for the removal of the cyanoethyl group and one C₁₋₄ alkyl group from the oligonucleotide compound of formula II. Particularly the task was to find reaction conditions which suppress or at least reduce unwanted side reactions, such as the formation of alkyl transfer impurities and of cyanoethyl (CNET) impurities.

It was found that the object could be achieved with the process for the production of a linear P-linked oligonucleotide bearing an alkyl phosphonate group at the 5'-terminus of the formula I 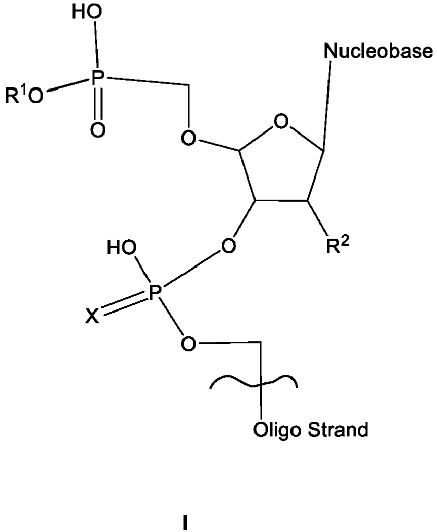 wherein R¹ is a C₁₋₄-alkyl group, R² is fluoro, hydroxyl or C₁₋₄-alkoxy, X is sulfur or oxygen, the term Nucleobase stands for an optionally modified adenine, cytosine, guanine or uracil and the term oligo strand stands for the remaining P-linked oligo nucleotide strand, and wherein the oligo strand comprises at least one uracil nucleobase,
which comprises the removal of the cyanoethyl group and one C₁₋₄ alkyl group from the oligonucleotide compound of formula II 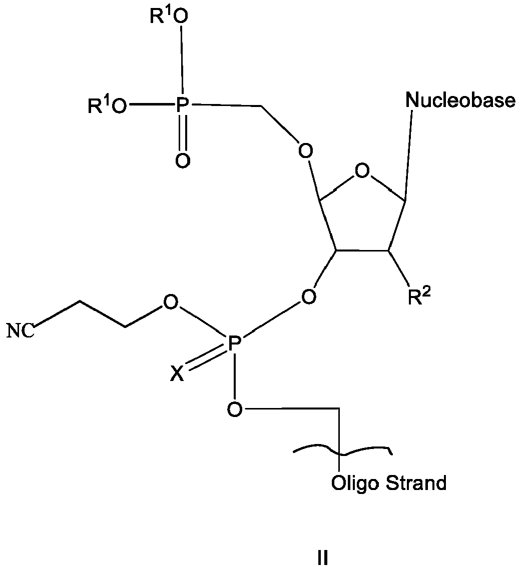 wherein R¹, R², X, the term Nucleobase and the term oligo strand is as above,
with a nucleophilic organic base in the presence of an organic solvent.

The following definitions are set forth to illustrate and define the meaning and scope of the various terms used to describe the invention herein.

The term C₁-₄-alkyl stands for a linear or branched alkyl group of 1 to 4 C-atoms. Representatives are methyl, ethyl, n-propyl, i-propyl, n-butyl, i-butyl or t-butyl. Preferable C₁-₄-alkyl group for the purpose of the present invention are methyl and ethyl, more preferably methyl.

The term C₁-₄-alkoxy stands for a linear or branched alkyl group of 1 to 4 C-atoms which is covalently bound to an oxygen atom. Representatives are methoxy, ethoxy, n-propoxy, i-propoxy, n-butoxy, i-butoxy or t-butoxy. Preferable C₁-₄-alkoxy group for the purpose of the present invention are methoxy and ethoxy, more preferably methoxy.

The term oligonucleotide as used herein is defined as it is generally understood by the skilled person as a molecule comprising two or more covalently linked nucleotides. For use as a therapeutically valuable oligonucleotide, oligonucleotides are typically synthesized as 10 to 40 nucleotides, preferably 10 to 25 nucleotides in length.

The oligonucleotides may consist of optionally modified DNA, RNA or LNA nucleoside monomers or combinations thereof.

The LNA nucleoside monomers are modified nucleosides which comprise a linker group or a bridge between C2' and C4' of the ribose sugar ring of a nucleotide. These nucleosides are also termed bridged nucleic acid or bicyclic nucleic acid (BNA) in the literature.

Optionally modified as used herein refers to nucleosides modified as compared to the equivalent DNA, RNA or LNA nucleoside by the introduction of one or more modifications of the sugar moiety or the nucleobase moiety. In a preferred embodiment the modified nucleoside comprises a modified sugar moiety, and may for example comprise one or more 2' substituted nucleosides and/or one or more LNA nucleosides. The term modified nucleoside may also be used herein interchangeably with the term "nucleoside analogue" or modified "units" or modified "monomers".

The DNA, RNA or LNA nucleosides are as a rule linked by a phosphodiester (P=O) and / or a phosphorothioate (P=S) internucleoside linkage which covalently couples two nucleosides together.

Accordingly, in some oligonucleotides all internucleoside linkages may consist of a phosphodiester (P=O), in other oligonucleotides all internucleoside linkages may consist of a phosphorothioate (P=S) or in still other oligonucleotides the sequence of internucleoside linkages vary and comprise both phosphodiester (P=O) and phosphorothioate (P=S) internucleoside.

The oligonucleotide constitution may be indicated from 5'-end (left) to 3'-end (right) using a three letter code with each nucleotide being described by three letters where
- the first letter defines the sugar moiety (f = 2'-fluoro-2'-deoxyribonucleotide, g = 2'-modified GalNAc ribonucleotide, m= 2'-*O*-methyl ribonucleotide, p = methylphosphonate-2'-*O*-methyl ribonucleotide isostere),
- the second letter defines the nucleobase (A=adenine, C=cytosine, G=guanine, U=uracil)
- the third letter defines the phosphor backbone (o= phosphodiester, s = phosphorothioate)

The term modified nucleobases include but are not limited to nucleobases carrying protecting groups and can be selected from tert.butylphenoxyacetyl, phenoxyacetyl, benzoyl, acetyl, isobutyryl or dimethylformamidino (see Wikipedia, Phosphoramidit-Synthese, https://de.wikipedia.org/wiki/Phosphoramidit-Synthese of March 24, 2016).

The principles of the oligonucleotide synthesis are well known in the art (see e.g. Oligonucleotide synthesis; Wikipedia, the free encyclopedia; https://en.wikipedia.org/wiki/Oligonucleotide synthesis, of March 15, 2016).

Larger scale oligonucleotide synthesis nowadays is carried automatically using computer controlled synthesizers.

As a rule, oligonucleotide synthesis is a solid-phase synthesis, wherein the oligonucleotide being assembled is covalently bound, via its 3'-terminal hydroxy group, to a solid support material and remains attached to it over the entire course of the chain assembly. Suitable supports are the commercial available macroporous polystyrene supports like the Primer support 5G from GE Healthcare or the NittoPhase^{®}HL support from Kinovate, or controlled pore glass supports like the nucleobase pre-loaded support from LGC.

As outlined above the oligonucleotide synthesis in principle is a stepwise addition of nucleoside residues to the 5'-terminus of the growing chain until the desired sequence is assembled as outlined above.

The backbone deprotection can be performed in accordance with the process of the present invention as outlined below. The subsequent cleavage from the resin can be performed with concentrated aqueous ammonia.

In a preferred embodiment the present invention comprises the process for the production of a linear P-linked oligonucleotide bearing an alkyl phosphonate group at the 5'-terminus of the formula Ia 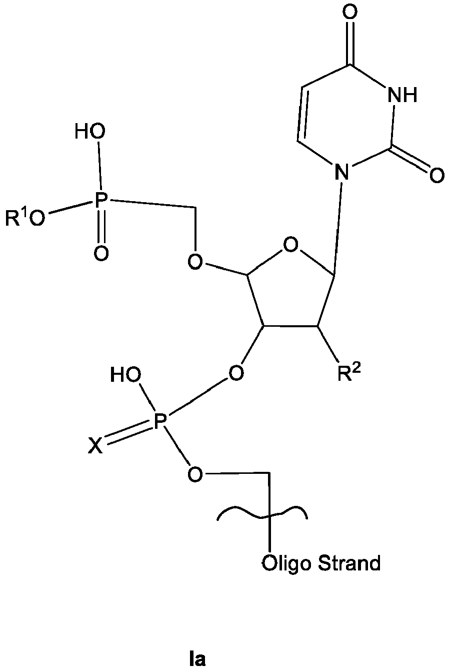 wherein R¹ is a C₁₋₄-alkyl group, R² is fluoro, hydroxyl or C₁₋₄-alkoxy, X is sulfur or oxygen and oligo strand stands for the remaining P-linked oligo nucleotide strand.

The starting compound of the process of the present invention, the oligonucleotide compound of formula II, can be prepared in accordance with the disclosure of the PCT International Publication WO 2018/045317.

In a preferred embodiment the oligonucleotide compound of formula II has the formula IIb

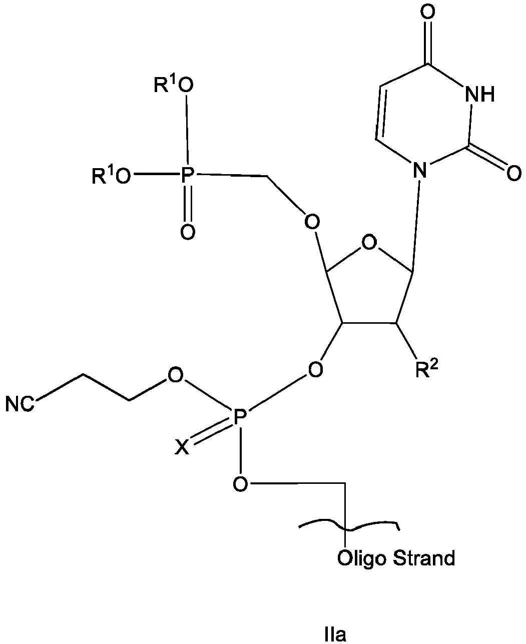

As outlined above R¹ is a C₁₋₄-alkyl group, R² is fluoro, hydroxyl or C₁₋₄-alkoxy. In a preferred embodiment R¹ is methyl and R² is fluoro, hydroxyl or methoxy. In a more preferred embodiment R¹ is methyl and R² is fluoro or methoxy.

The nucleophilic organic base typically is an organic amine which can be characterized by its nucleophilicity in accordance with the Mayr nucleophilicity scale: J. Phys. Org. Chem. 2008, 21, 584-595) and its pKa in accordance with Wikipedia, Acid dissociation constant, https://en.wikipedia.org/wiki/Acid_dissociation_constant of October 24, 2021).

Usually the nucleophilic organic base has nucleophilicity of higher than 15, preferably between 15 and 30, more preferably between 16 and 25.

The pKa value of suitable nucleophilic organic bases base (protonated organic base) is less than 10.5, preferably between 6 and 10.5., more preferably between 8 and 10.5.

Preferred nucleophilic organic bases are tertiary amines which can be selected from morpholine, N,N-dimethylethylamine, N-methyl-pyrrolidine or from (1,4-diaza-bicyclo[2.2.2]octane.

Preferred nucleophilic organic base is (1,4-diazabicyclo[2.2.2]octane) (DABCO).

The process requires the presence of an organic solvent, which can be selected from acetonitrile, pyridine and toluene or from mixtures thereof. Preferred organic solvent is acetonitrile.

The concentration of the nucleophilic organic base in the organic solvent is as a rule selected in the range of 5% (w) and 100% (w), preferably 10 % (w) to 50 % (w), more preferably 15 % (w) to 25 % (w).

The amount of the nucleophilic organic base is typically applied in a range of 1.5 CV to 30.0 CV.

The solution of the nucleophilic organic base in the organic solvent is usually delivered in a time range of 10 min to 6 hours, preferably 20 min to 4 h.

A flow rate in the range of 0.1 CV/min to 2.0 CV/min, preferably 0.1 CV/min to 0.5 CV/min was found to be practicable.

After complete delivery, the solution of the nucleophilic organic base in the organic solvent can be recycled over the synthesis column for a time between 60 min and 4 hours, preferably 90 min to 120 min. This is a further preferred embodiment as it allows, if required, to prolong the reaction time without the need to add fresh organic base.

As a typical example, 3.75 CV of the nucleophilic organic base in acetonitrile is charged to the column over 30 min followed by recycling over the column for 90 min at 0.125 CV/min.

As mentioned above the object of the present intention was suppressing or at least reducing unwanted formation of alkyl transfer impurities and of cyanoethyl (CNET) impurities because the alkyl group (R¹) has a tendency to alkylate uracil nucleobases in the neighborhood of the alkylphosphonate nucleotide, which results in so called alkyl transfer impurities as illustrated in the oligo structure of formula III below with a methylated uracil nucleobase.

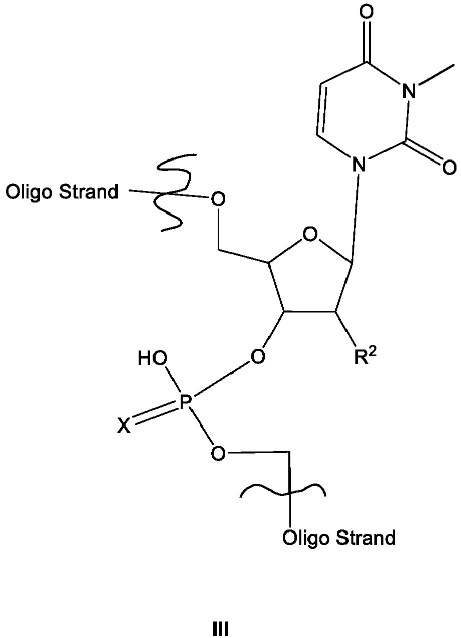

These impurities cannot be significantly depleted in the subsequent downstream processing steps.

In view of the fact that the alkyl transfer impurities or preferably the methyl transfer impurities affect uracil nucleobases the oligo strand in the oligonucleotide compound of formula II or IIa and in the resulting linear P-linked oligonucleotide of the formula I or Ia contain at least one uracil nucleobase.

Since the tendency to alkylate the uracil nucleobase and with that the alkyl transfer impurities increase the closer the uracil nucleobases are located on the oligo strand to the 5' terminus, the term neighborhood means that the uracil nucleobases are typically within the 2^{nd} to the 20^{th} position, preferably within the 2^{nd} to the 10^{th} position, more preferably within the 2^{nd} and the 6^{th} position of the oligo strand, counted from the 5' terminus.

The process of the present invention as a further embodiment therefore comprises performing the process under conditions that the level of alkyl transfer impurities, expressed as "sum of N+ alkyl impurities", in the linear P-linked oligonucleotide of formula I is below 4.0%, below 3.0%, below 2.0%, below 1.0%, or most preferred below 0.5%. The % values are "% area" determined from the %area of the UV peaks, which is corrected by MS intensity of the N+ alkyl impurity group.

In a preferred embodiment the N+alkyl impurities are N+methyl impurities.

The process of the present invention as a further embodiment also comprises performing the process under conditions that the level of cyanoethyl (CNET) impurities, expressed as "sum of CNET impurities", in the linear P-linked oligonucleotide of formula I, is below 2.0%, below 1.0% or below 0.5%. The % values are "% area" determined from the %area of the UV peaks, which is corrected by MS intensity of the CNET impurity group.

These values can be achieved and measured at the crude oligonucleotide stage, i.e. for the oligonucleotide obtained after cleavage and deprotection and before any downstream processing like purification or ultrafiltration is applied.

By way of illustration the oligonucleotide can be selected from:
pUs.fUs.fAs.mUo.fUo.mGo.fUo.fGo.mAo.fGo.mGo.fAo.mUo.fUo.mUo.fUo.mUo.mGo.f Uo.mCs.mGs.mG
wherein the oligonucleotide constitution is indicated from 5'-end (left) to 3'-end (right) using a three letter code with each nucleotide being described by three letters where
- the first letter defines the sugar moiety (f = 2'-fluoro-2'-deoxyribonucleotide, g = 2'-modified GalNAc ribonucleotide, m= 2'-*O*-methyl ribonucleotide, p= methylphosphonate-2'-*O*-methyl ribonucleotide isostere),
- the second letter defines the nucleobase (A=adenine, C=cytosine, G=guanine, U=uracil)
- the third letter defines the phosphor backbone (o= phosphodiester, s = phosphorothioate)

The compounds disclosed herein have the following nucleobase sequences
SEQ ID No. 1: UUAUUGUGAGGAUUUUUGUCGG

### Examples

Abbreviations:
AC₂O = acetic acid anhydride
ETT = 5-ethylthiotetrazol
Bz = benzoyl
CNET = cyanoethlyl
DABCO = 1,4-diazabicyclo[2.2.2]octane
DCA = dichloroacetic acid
DEA = diethylamine
DNA = 2'-deoxyribonuleotide
DMEA = N'N dimethylethylamine
DMT = 4,4'-dimethoxytrityl
CV = column volume
MeCN = acetonitrile
NA = not applicable
NMI = N-methyl imidazole
NMP = N-methyl-pyrrolidine
PhMe = Toluene
TBA = tert-butylamine
PhMe = Toluene

### Example 1.

### Synthesis of pUs.fUs.fAs.mUo.fUo.mGo.fUo.fGo.mAo.fGo.mGo.fAo.mUo.fUo.mUo.fUo.mUo.mGo .fUo.mCs.mGs.mG

Wherein the oligonucleotide constitution is indicated from 5'-end (left) to 3'-end (right) using a three letter code with each nucleotide being described by three letters where
- the first letter defines the sugar moiety (f = 2'-fluoro-2'-deoxyribonucleotide, g = 2'-modified GalNAc ribonucleotide, m= 2'-*O*-methyl ribonucleotide, p=methylphosphonate-2'-*O*-methyl ribonucleotide isostere),
- the second letter defines the nucleobase (A=adenine, C=cytosine, G=guanine, U=uracil)
- the third letter defines the phosphor backbone (o= phosphodiester, s = phosphorothioate)

The title compound was produced by standard phosphoramidite chemistry on solid phase at a scale of 2.62 mmol using an AKTA Oligopilot 100 and preloaded polystyrene solid support (NittoPhase LH preloaded 358).

The following phosphoramidites have been used in each cycle:

| **Cycle** | **P-amidite** | **Cycle** | **P-amidite** |
|---|---|---|---|
| 1 | mG(iBu) | 12 | fG(iBu) |
| 2 | mC(Ac) | 13 | mA(Bz) |
| 3 | fU | 14 | fG(iBu) |
| 4 | mG(iBu) | 15 | fU |
| 5 | mU | 16 | mG(iBu) |
| 6 | fU | 17 | fU |
| 7 | mU | 18 | mU |
| 8 | fU | 19 | fA(Bz) |
| 9 | mU | 20 | fU |
| 10 | fA(Bz) | 21 | pU |
| 11 | mG(iBu) | | |

In general 2.0 equiv of the phosphoramidites were employed. All reagents were used as received from commercially available sources and reagent solutions at the appropriate concentration were prepared (see details below). Cleavage and deprotection was achieved using ammonium hydroxide to give the crude oligonucleotide.

### Standard Reagent Solutions

| | |
|---|---|
| Deblock | 3 vol% DCA in toluene |
| Phosphoramidites | 0.2 M in acetonitrile for mA(Bz), mC(Ac), mG(iBu), fA(Bz), fC(Ac), fG(iBu), fU, pU 0.2 M in acetonitrile/toluene (85:15, v/v) for mU |
| Activator | 0.6 M ETT in acetonitrile |
| Thiolation | 0.2 M Xanthanhydride in pyridine |
| Cap A | NMI / 2,6-lutidine / acetonitrile: 20/30/50 |
| Cap B | 20 vol% acetic anhydride in acetonitrile |
| Backbone deprotection | See experiments |
| Cleavage and Deprotection | 28-32% aqueous ammonium hydroxide at 35°C |

The crude solution from the cleavage & deprotection step was concentrated in vacuo to remove excess ammonia. The concentrated solution was lyophilized to provide the crude oligonucleotide as a solid. The pale yellow solid was sampled and submitted to LC-UV-MS analysis. Impurities were grouped according to their assigned structure. The sum of all N+Methyl impurities and the sum of all CNET impurities were used for the analysis of the process parameters.

### Example 2

### Backbone deprotection examples

The organic amines used have the pKa and nucleophilicity values listed in the table below. The nucleophilicity values can be found in Mayr's Database of Reactivity Parameters (https://www.cup.lmu.de/oc/mayr/reaktionsdatenbank/fe/showclass/40) and the pKa values in "Correlation of the Base Strengths of Amines, J. Am. Chem. Soc. 1957, 79, 20, 5441-5444". The pKa of DABCO is referenced in the "Basicity of 1,s-bis(dimethylamino)-naphthalene and 1,4-diazabicyclo[2.2.2]octane in water and dimethylsulfoxide, Can. J. Chem. 1987, 65, 996.

| Amine | pKa | Nucleophilicity |
|---|---|---|
| DABCO | 8.82 | 18.80 |
| DEA | 10.98 | 15.10 |
| DMEA | 9.99 | N/A |
| Morpholine | 8.36 | 15.65 |
| NMP | 10.46 | 20.59 |
| TBA | 10.45 | 12.35 |
| TEA | 10.65 | 17.10 |

Backbone deprotection was performed using various solutions of organic amines in toluene, pyridine or acetonitrile as outlined in the table below.

| Example | Nucleophilic organic base (Nob) | Solvent | Concentration Nob in solvent [weight 1%] | Sum of N+methyl impurities [area%] | Sum of CNET impurities [area%] | Yield [%] |
|---|---|---|---|---|---|---|
| 2a) comparison | TBA | MeCN | 18.1 | 5.6 | 0.5 | 72 |
| 2b) comparison | TBA | MeCN | 18.1 | 8.3 | 0.9 | 70 |
| 2c) comparison | DEA | MeCN | 18.4 | 5.2 | 0.3 | 71 |
| 2d) comparison | TEA | MeCN | 18.8 | 4.2 | 0.2 | 72 |
| 2e) | Morpholine | MeCN | 24.1 | 1.7 | 0.1 | 71 |
| 2f) | Morpholine | MeCN | 45.8 | 1.6 | 0.1 | 54 |
| 2g) | Morpholine | - | 100 | 0.9 | 0.1 | 59 |
| 2h) | Morpholine | Pyridine | 20.2 | 2.5 | 0.1 | 67 |
| 2i) | Morpholine | Toluene | 22.3 | 2.3 | 0.2 | 70 |
| 2j) | DABCO | MeCN | 20 | 0.3 | 0.1 | 70 |
| 2k) | DABCO | MeCN | 20 | 0.3 | 0.1 | 70 |
| 21) | DMEA | MeCN | 17.7 | 2.2 | 0.1 | 72 |
| 2m) | NMP | MeCN | 20.7 | 1.7 | 0.3 | 70 |

The process parameters are listed the separate table below.

| Example | Nucleophilic organic base (Nob) | Solvent | Amount of Nob in solvent [CV] | Delivery time [min] | Recycling time [min] | Yield [%] |
|---|---|---|---|---|---|---|
| 2a) comparison | TBA | MeCN | 5.00 | 45 | - | 72 |
| 2b) comparison | TBA | MeCN | 26.67 | 240 | - | 70 |
| 2c) comparison | DEA | MeCN | 26.67 | 240 | - | 71 |
| 2d) comparison | TEA | MeCN | 26.67 | 240 | - | 72 |
| 2e) | Morpholine | MeCN | 26.67 | 240 | - | 71 |
| 2f) | Morpholine | MeCN | 26.67 | 240 | - | 54 |

| | | | | | | |
|---|---|---|---|---|---|---|
| 2g) | Morpholine | - | 5.00 | 45 | 195 | 59 |
| 2h) | Morpholine | Pyridine | 26.67 | 240 | - | 67 |
| 2i) | Morpholine | Toluene | 26.67 | 240 | - | 70 |
| 2j) | DABCO | MeCN | 26.67 | 240 | - | 70 |
| 2k) | DABCO | MeCN | 3.75 | 30 | 90 | 70 |
| 21) | DMEA | MeCN | 26.67 | 240 | - | 72 |
| 2m) | NMP | MeCN | 26.67 | 240 | - | 70 |

## Claims

1. Process for the production of a linear P-linked oligonucleotide bearing an alkyl phosphonate group at the 5'-terminus of the formula 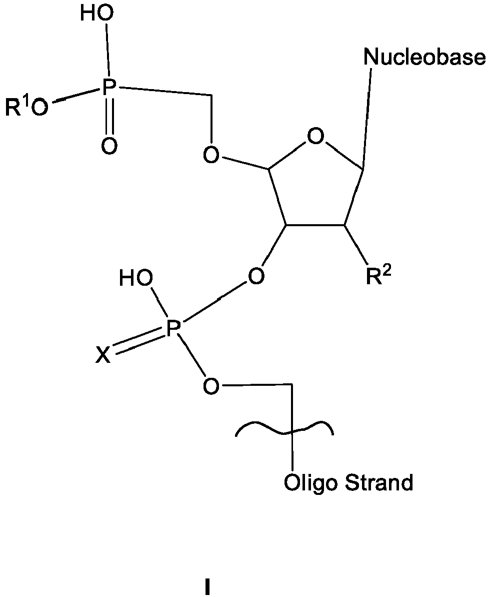 wherein R¹ is a C₁₋₄-alkyl group, R² is fluoro, hydroxyl or C₁₋₄-alkoxy, X is sulfur or oxygen, the term Nucleobase stands for an optionally modified adenine, cytosine, guanine or uracil and the term oligo strand stands for the remaining P-linked oligo nucleotide strand, and wherein the oligo strand comprises at least one uracil nucleobase,
comprising the removal of the cyanoethyl group and one C₁₋₄ alkyl group from the oligonucleotide compound of formula II 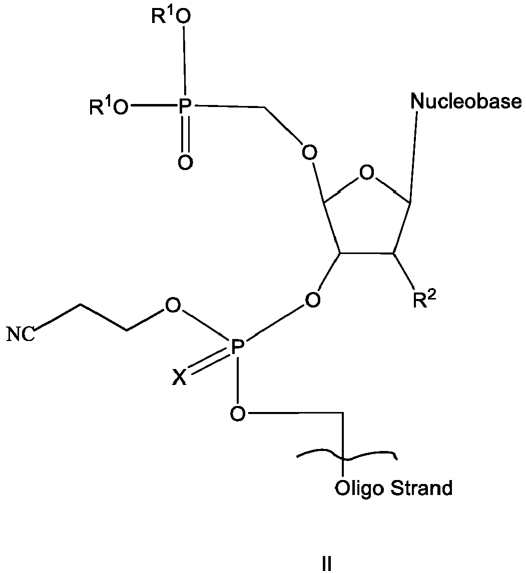 wherein R¹, R², X, the term Nucleobase and the term oligo strand is as above, with a nucleophilic organic base in the presence of an organic solvent.

2. Process of claim 1, wherein R¹ is methyl.

3. Process of claim 1 or 2, wherein R² is fluoro or methoxy.

4. Process of anyone of claims 1 to 3, wherein the Nucleobase is uracil.

5. Process of anyone of claims 1 to 4, wherein the nucleophilic organic base has a nucleophilicity of higher than 15, preferably between 15 and 30, more preferably between 16 and 25.

6. Process of anyone of claims 1 to 5, wherein the nucleophilic organic base has a pKa of the protonated organic base of less than 10.5, preferably between 6 and 10.5, more preferably between 8 and 10.5.

7. Process of anyone of claims 1 to 6, wherein the nucleophilic organic base is a tertiary amine selected from morpholine, N,N-dimethylethylamine, N-methyl-pyrrolidine or from (1,4-diazabicyclo[2.2.2]octane).

8. Process of anyone of claims 1 to 7, wherein the nucleophilic organic base is (1,4-diazabicyclo[2.2.2]octane) (DABCO).

9. Process of anyone of claims 1 to 8, wherein the organic solvent is selected from acetonitrile, pyridine and toluene or from mixtures thereof.

10. Process of anyone of claims 1 to 9, wherein the organic solvent is acetonitrile.

11. Process of any one of claims 1 to 10, wherein the concentration of the nucleophilic organic base in the organic solvent is selected in the range of 5% (w) and 100% (w), preferably 10 % (w) to 50 % (w), more preferably 15 % (w) to 25 % (w).

12. Process of any one of claims 1 to 11, wherein the amount of the nucleophilic organic base is applied in a range of 1.5 CV to 30.0 CV and the flow rate is selected in the range of 0.1 CV/min to 2.0 CV/min, preferably 0.1 CV/min to 0.5 CV/min.

13. Process of any one of claims 1 to 12, wherein the process is performed under conditions that the level of alkyl transfer impurities, expressed as "sum of N+ alkyl impurities", in the linear P-linked oligonucleotide of formula I, is below 4.0%, below 3.0%, below 2.0%, below 1.0%, or below 0.5%.

14. Process of any one of claims 1 to 13, wherein the process is performed under conditions that the level cyanoethyl (CNET) impurities, expressed as "sum of CNET impurities", in the linear P-linked oligonucleotide of formula I, is below 2.0%, below 1.0% or below 0.5%.
